# NEW EUROPEAN PATENT SPECIFICATION

(11) **EP 0 171 150 B2**
(45) Date of publication and mention of the opposition decision: **02.09.1998**
(45) Mention of the grant of the patent: 25.03.1992
(21) Application number: 85304197.8
(22) Date of filing: 12.06.1985
(51) Int. Cl.: G01N 33/543, C12Q 1/28, C12Q 1/54

(54) **Method and apparatus for assaying with optional reagent quality control**
Verfahren und Gerät für Testverfahren mittels fakultativ für Qualität kontrollierter Reagenzien
Méthode et dispositif pour essais utilisant des réactifs de qualité contrôlée facultativement

(30) Priority: 12.06.1984 US 619739
(43) Date of publication of application: 12.02.1986
(73) Proprietor: Orgenics Ltd., Yavne 70650 (IL)
(72) Inventor: Herzberg, Max, Moshav Sataria 73772 (IL); Fish, Falk, Tel Aviv, 69499 (IL)
(74) Representative: Clifford, Frederick Alan

(56) References cited:
- GB-A- 1 442 291
- US-A- 3 941 876
- US-A- 4 299 916
- US-A- 4 558 013
- DR. OTTO-ALBRECHT NEUMÜLLER, "Römpps Chemie-Lexikon", 8th edition, vol. 3, 1983, Frankh'sche Verlagsbuchhandlung, Stuttgart (DE)
- MAST Inhalant Profile, Western Region. (Brochure 1983)

## Description

### BACKGROUND OF THE INVENTION

### 1. Field of the Invention

The present invention relates to a method and apparatus for quantitatively and/or qualitatively assaying an analyte.

### 2. Description of Material Information and Related Materials

Solid phase immuno-assays offer the most specific and sensitive methods for detecting and measuring an unknown material (analyte) in a given sample. While the actual use of these methods in the field is quite limited at present, various versions of solid phase assays have been developed and improvements have been proposed such that the earlier fluid phase immuno-assays (eg gel diffusion, precipitation, agglutination) will probably be replaced by a new generation of immuno-assays. Solid phase assays may be more complicated and require more elaborate and complicated technology but they offer higher sensitivity and are better adapted for quantitative work. The present state of the art and the applicability of solid phase immuno-assays are extensively reviewed in US Patent Nos 3,654,090 and 4,299,916.

Briefly, present methods are capable of measuring an unknown analyte provided it is either an antibody to a known antigen or an antigen, ie, a material to which a specific antibody can bind.

Depending on the nature of the unknown, ie, whether it is an antibody or an antigen, the specific counterpart (receptor) is affixed onto a solid surface such as the inner wall of a test tube, a dip-stick or beads. The analyte then binds to its solid phase receptor. The presence of the bound analyte and its quantity can then be determined by a labelled molecule (probe) which is either the analyte itself (competitive assay) or an antibody to the analyte ("sandwich" assay). The label can be radioactive (Radio Immuno-assay), enzymatic (Enzyme immuno-assay) or fluorescent (Fluorescence immuno-assay). The solid phase principle facilitates the separation of unbound labelled and unlabelled components of the assay system (antigens and/or antibodies).

Whilst sensitivity of solid phase immuno-assay can be tailored to the specific case, and specificity can be easily obtained, assays require certain improvements to make them more reliable, usable and portable. Thus, it would be desirable to:
a) Simplify and stabilize reagents, so that the assay components can be stored inexpensively and for long periods of time.
b) Provide an instantaneous, built-in quality control monitoring system for each of the steps involved since materials can become stale and steps may be improperly performed, thus affecting the final result.
c) Perform differential diagnosis of infectious diseases where a plurality of unknowns can be screened in a single assay. Such a diagnosis would preferably be performed using a single apparatus with a minimum of steps.
d) Provide an apparatus with which untrained field staff may perform the assays and analyze the results obtained by visual inspection. To accomplish this the assay kits should be storage stable and simple to use. Such kits should include a minimal number of components and should lend themselves to easy filing and record keeping of the unknown, and standard values.

### SUMMARY OF THE INVENTION

It is an object of the invention to provide an assay technique and apparatus which is compact and effective and which lends itself to careful quality control by the user in a manner which minimizes risk of error due to false positive or negative results.

It is a further object of the invention to provide an assay technique and kit in which the effectiveness of all of the constituents, and timing of the assay may be verified by the user at will.

It is yet another object of the invention to provide an assay kit in which semi-quantitative tests may be performed by visual inspection, thereby avoiding the necessity of expensive optical measuring apparatus. Yet more accurate quantitative readings are possible using optical measurement apparatus.

Another feature of the invention allows for a kit in which various reagents are preserved in a dried, or semi-dried state, and need only be re-hydrated as needed, just prior to use. The use of such a kit, together with means for insuring reagent quality during the assay, thus provides a very powerful diagnostic tool.

According to the present invention there is provided a method for detecting at least one analyte in a single liquid solution suspected of containing more than one analyte, said method comprising the steps of:
(a) providing a solid support which is water insoluble and flexible and made from material selected from the group of polyvinyl, polystyrene, cellulose, nylon or glass, shaped in a form of flat, rectangular or round sheet, a rod, a stick or a cylinder;
(b) attaching and selectively positioned on said support at least one receptor for said at least one analyte, said receptor being selected from the group consisting of proteins, immunoglobins, nucleic acids or polysaccharides, carbohydrates, lipids;
(c) attaching a plurality of control spots on a pre-designated area of said solid support for monitoring a plurality of reactions of said analyte - detecting method ;
(d) swabbing said liquid solution on said support (c), or dipping said support (c) into said liquid solution, wherein said liquid solution contains at least one analyte selected from the group consisting of proteins, nucleic acids, carbohydrates, immunoglobins, polysaccharides, lipids to form receptor-analyte conjugate reaction;
(e) contacting.said receptor-analyte conjugate reaction (d) with a signal generating reagents to determine the presence and/or extent of said at least one analyte, wherein said signal generating reagents are labelled probes selected from antibodies, antigens, enzymes, carbohydrates, polysaccharides, antigens and nucleic acids, proteins and lipids;
said support further comprising standard spots thereon for establishing standard values which are compared with the results observed.

Receptors may naturally be adapted for attachment to the solid support. In certain instances the receptor and/or support may have to be modified for attachment. Such a technique is disclosed, eg, in USP 4,299,916 wherein linking groups are used to attach receptors onto the surface of the solid support. The attachment technique used preferably provides a minimum of background noise and does not interfere with the activity of the receptor vis-a-vis the analyte or the probe.

To provide binding site amplification on the support, the solid support may be treated with a liquid solution before the receptors have been bound to add additional binding sites. Such a technique is disclosed in Fish and Ziff, Arth Rheum 24:534, 1981, the disclosure of which is incorporated herein by reference thereto. The solid support may be contacted with the sample by dipping it into a liquid solution of the analyte, or by dropping, streaking or swabbing the sample across the receptor locations on the support. Where testing for multiple analytes, the solid support may be contacted with the sample during a sequence in which each contact is performed under physicochemical conditions most favorable for each receptor-analyte binding pair.

The solid support may be washed after each step, if desired, the first wash occurring after the support has contacted with the sample. The wash solution is preferably 0.5M NaCI. The solution may also contain a detergent, eg, BRIJ 35.

Where the probe is labelled, the labelled probe may be a labelled receptor or labelled antibody to the analyte such that a sandwich is formed. By way of example, the labelled probe may be an antibody against the immunoglobulin type of the analyte in assays to detect and measure antibody analytes in the sample.

The label itself may be radioactive or enzymatic. Enzymatic labels usable according to the invention are eg, peroxidase or glucose oxidase, both of which provide a color reaction, although other chromogenic enzyme substrate combinations may quite obviously be used.

According to a preferred aspect of the invention, the label is a substance which converts soluble, colorless chromogen into an insoluble dye, which precipitates at the site of the bound label to develop a color reaction. Alternatively, the label may convert insoluble colorless chromogen into soluble colored dye at the site of the bound label to allow for observation and measurement.

Where the analyte is a nucleic acid, a labelled antibody may be used to detect and measure nucleic acids in the samples. By way of example, the labelled antibody may be anti-double-stranded DNA antibody.

Where the analyte is a nucleic acid it may previously have been chemically modified, in which case the labelled antibody may be an antibody to the chemical modification. The nucleic acid may, according to one embodiment, be modified by picryl sulfonate according to a technique disclosed, for example, in Kiefer, "immunological Methods", edited by Lefkovitz and Pernis, Academic Press 1979, pp 137-150.

According to yet another embodiment a nucleic acid may be utilized as an unlabelled probe as is disclosed, for example, in International Application PC WO03/01459. The nucleic acid may be chemically modified, and be capable of annealing with the bound analyte. A labelled anti-probe is then utilized which is an antibody to the chemical modification. The unlabelled probe may be identical to the bound receptor prior to the chemical modification. The unlabelled probe is preferably modified by picryl sulfonate. Where desired, the unlabelled probe can be hydrolyzed into smaller pieces of nucleic acid, prior to being chemically modified.

According to the invention, a quality control system is provided for monitoring at least some, if not all of the assay components, for proper activity, as well as controlling exposure times and conditions.

The quality control system may include:
(a) control for the activity of the dye-substrate system:
(b) control of the receptor;
(c) control for the activity of the probe: and
(d) control for sufficient incubation times.

The various components of the assay may be monitored for proper activity by binding control materials to specific areas on the solid support. The control substances may generally be selected from the group of proteins, nucleic acids, carbohydrates, polysaccharides, lipids, and combinations thereof.

Thus, an enzyme which will complex and react with the dye-substrate development system may be bound to the support to provide control for the activity of the dye-substrate system.

Additionally or alternatively, a material corresponding to analyte may be bound to the support to test for proper probe-analyte activity. After exposure and development of the signals provided by the control areas and the receptor areas, the resulting signal is compared to the signal produced by the bound analyte from the sample. A comparison defines the presence and/or extent of the presence of the bound analyte. This technique also serves as a control with respect to the activity of the probe-containing solution since if it is not sufficiently active it will provide an inadequate color reaction.

Also, the activity of the label of the labelled probe or labelled anti-probe may also be controlled by contacting the probe with specific areas on the solid support to which is bound a material selected from the group consisting of: avidin, biotin, an antibody or any receptor with which the probe or anti-probe label complexes to provide a measurable signal.

The presence of the bound probe (or anti-probe) may be measured semi-quantitatively, such as by observation, or quantitatively with conventional instrumentation.

The invention is further directed to a container for use in conjunction with assaying techniques in which the various reagents are provided within the container in liquid, dried, or semi-dried form. Where necessary, the reagents are reconstituted just prior to use. The container is provided with individual compartments in which the various steps of the assay are individually performed. Thus, each of the compartments would contain different reagents corresponding to the different steps of the assay.

Thus, according to the present invention there is provided an apparatus according to claim 17.

Each of the individual compartments may be provided with an orifice through which liquid may be injected to reconstitute the reagent. The orifice may be covered with a rubber septum at the bottom of each of the individual compartments to prevent unnecessary exposure to humidity. The compartments are likewise each provided with an opening in the top which is covered by a rubber strip having an opening in it which is normally closed but which allows for insertion and removal of the support and may be eg, longitudinal, circular, etc. Where it is longitudinal, the opening of the rubber strip parallels the interior walls of each of the individual compartments. The length of the opening is at least as long as the width of the solid support to allow for insertion and removal. Each of the individual compartments is filled with reagents and sealed separately with adhesive tape.

The solid support is dipped into the appropriate individual compartments for the required length of time, and pulled out of each individual compartment through the openings topped with the rubber cover strip having its longitudinal slit therein. The lips of the rubber strip facilitate more complete removal of the reagent from the solid support by a wiping action. The lips are closed enough to provide the desired wiping action.

### BRIEF DESCRIPTION OF THE DRAWINGS

Figure 1 illustrates one possible solid flexible support according to the invention;
Figure 2 illustrates a container which may be used with the technique of the invention: and
Figure 3 illustrates a developed rod according to the invention.

### DESCRIPTION OF PREFERRED EMBODIMENTS

The invention relates to a method and apparatus capable of quantitatively and qualitatively analyzing an analyte in a manner which allows for improved quality control.

The inventive assay system finds particular application when analyzing for a plurality of analytes from a single sample, ie, a multi-analyte assay. This multiple analysis capability provides the advantage of analyzing for more than a single analyte in cases where more than a single analyte is suspected. This is of particular interest where attempting to perform a differential diagnosis, and where multiple assays might normally be indicated.

The invention is particularly valuable in connection with differential diagnosis of infectious diseases. According to the present state of the art. immuno-diagnosis of such diseases is possible only after the organism has been previously isolated, cultured, and identified by microbiological, virological, and or biochemical means. Existing immunodiagnostic assays detect only "group antigens", such that the technique is able to specify only the group or strain to which the isolated bacterium belongs. Although such tests are of importance in epidemiological studies. they are not sufficiently specific for non-pathogenic variance of the same bacterium.

The technique of the invention makes it possible to perform a differential assay for a plurality of analytes in a sample.

For example. although the bacterium Escherichia Coli is a normal inhabitant of the human and animal intestine, at times, it can become pathogenic. When pathogenic, the pathogenic variant may carry the same group antigens as the normal variety. The most specific way to distinguish and identify pathogenic organisms is by their "virulence factors". These factors are structures or molecules which enable the bacterium to invade the host and cause disease. Some of these factors are exo-toxins, hemolysins, and adherence factors.

Table I lists certain pathogenic bacteria and some of their specific virulence factors which may serve as antigens in the multi-analyte differential diagnosis of infectious diseases according to the invention. Table I is, of course. given by way of illustration only and is intended to be neither conclusive nor limiting:

**TABLE I**

| Bacteria | Virulence Factors |
|---|---|
| Bacillus anthracis | Edema producing toxin complex |
| Bordetella pertussis | Pertussis toxin |
| Corynebacterium diphtheriae | Diphtheria toxin |
| Escherichia coli | Heat labile enterotoxin |
| Staphylococcus aureus | Exfoliating toxin; Enterotoxin |
| Streptococcus pyogenes | Erythrogenic toxin |

Differential detection is also useful with nucleic acid analytes. In this instance, a complementary receptor to be affixed to the substrate may be a complementary strand of nucleic acid. Where a single-stranded nucleic acid is the analyte, it can bind to the receptor without any prior treatment. Where the analyte is a double-stranded nucleic acid, denaturation of the analyte is necessary before exposing the analyte to the receptors.

Table II lists certain instances in which differential detection of nucleic acids may be useful, thus enhancing the value of the technique of the present invention.

**TABLE II**

| | | |
|---|---|---|
| 1. | Viral infections | detection of latent infection (Herpes, varicella-zoster. EBV); encephalomyelitis: measles or Rubella (differential diagnosis) |
| 2. | Hereditary diseases | early diagnosis (in parents and fetus). |

Table III is a partial list of groups of symptomatically similar diseases in which the detection of antigen and or antibody, can be helpful in differential diagnosis. Of course, the listing is not intended to be limiting or all-inclusive

**TABLE III**

| Syndrome | Etiologic Agents |
|---|---|
| Phyaryngitis & Tonsilitis (sore throat) | Strep. pyogenes, C. diphtheriae, N. gonorrheae, N. meningitidis, H. |
| | |
| influenza | B. Adenovirus, Herpes, Coxackie, Epstein Barr Virus |
| | |
| Otitis Media | Strep. pneumoniae, H. influenza, Strep. pyogenes, Staph aureus, Proteus, Pseudomonas |
| | |
| Influenza | Various hemagglutinin and cialidase types |
| | |
| Stomatitis | Differential diagnosis between Herpes and others needed to avoid use of steroids |
| | |
| Meningitis | H. influenza, N. meningitidis, Strep. pneumoniae, S. aureus, Cryptococcus neoformans, Herpes simplex |
| | |
| Parotitis | Mumps, Influenza, Parainfluenza, Staph, aureus, Strep. viridans, pneumococci, E. coli Haemophilus |
| | |
| Pertussis | B. pertussis, B. parapertussis, Adenovirus |
| | |
| Pneumonia | Strep, pneumoniae (= Pneumococcus) Strep. hemolyticus, Strep. pyrogenes, Staph. aureus, N. meningitidis, Klebsiella pneumoniae, E. coli, P. aeruginosa, H. influenza, Mycoplasma pneumoniae, Legionella pneumophila, Misc. viruses |
| | |
| Diarrhea | Shigella sp., Salmonella sp, E. coli, Cl. perfrigens, Vibrio parahemolyticus, Vibrio cholera, Campylobacter fetus, Yersinia enterocolitica, Rotavirus |
| | |
| Urinary Tract | E. coli, Proteus sp., |
| Infections | Aerobacter sp., Strep. faecalis, Staph. aureus, Pseudomonas sp., others |

The inventive technique also permits the concomitant assay of both antigens and antibodies, on a single substrate from the same sample. Such a technique is useful in the diagnosis of infectious diseases where an excess of antibody signifies the convalescent stage of the illness, and signifies that chemotherapy is no longer indicated.

The following are examples of antigen - antibody systems in which both members may be assayed according to the invention.

**TABLE IV**

| |
|---|
| Newcastle Disease |
| Whooping cough |
| Measles |
| Influenza |
| Pneumonia |

As was noted above, other analytes may be detected by detecting the following resultant receptor-analyte couples using the technique of the present invention: hormones and their receptors; various drugs, chemicals and vitamins, and their receptors; neurotransmitters and opiates and their receptors; enzymes and their substrates, and toxins and their receptors.

For purposes of the invention, the term "receptor" is used in its broadest sense and merely refers to any substance capable of complexing and/or affixing to an analyte in a manner which allows for subsequent use or treatment of the affixed material, preferably in a manner which facilitates its identification, and/or quantification, eg, commonly used receptors include proteins.

The system of the invention is useful in analyzing for the presence of virulence factors, eg, exotoxins. adherence factors, hemolysins; nucleic acids; hormones; drugs; chemicals; vitamins; neurotransmitters: opiates; enzymes: toxins; etc.

It is a preferred aspect of the invention that the assay be performed using a solid support having a plurality of receptors thereon, each of which is capable of binding a different analyte thereto. The solid support is insoluble in the solution being analyzed and is preferably sufficiently flexible to provide for ease of manipulation.

The support of the invention may assume a variety of configurations and may be round and flat, card-like, eg, square or rectangular and flat, tubular, a rod, a stick, cylinder, etc. The support used may obviously be any material capable of maintaining its general configuration during use. A preferred configuration according to the invention is a support in the form of a sheet which may be circular, but which is most preferably rectangular. Such a substrate may be formed of polyvinyl, polystyrene, or high-impact polystyrene, although other materials may quite obviously be used. The shape of the support, as well as the material of which it is made, may quite obviously vary depending upon the circumstances of use.

According to the invention a plurality of receptors, are bound to the solid surface of the support. The choice of the number and types of receptors to be used depends on the anticipated analytes. A plurality of different receptors, each specific to a different analyte will thus be used.

The different receptors may be bound to the support in a variety of ways. Immunoglobulins (antibodies) and many protein antigens, will spontaneously bind to various plastics eg, polystyrenes and polyvinyls, as well as other polymeric materials, eg, celluloses and nylons as well as to glass fibers. High-impact polystyrene can be an especially convenient solid support which allows for good binding of protein antigens and low background activities at later stages of the assay. Additionally, the support may be formed of a material which is coated with a layer of another material or polymer such as nitrocellulose for purposes of increasing the surface area of the support.

Single-stranded nucleic acids also bind spontaneously to the materials recited in the previous paragraph. Double stranded nucleic acids do not bind to any significant extent to such surfaces such that when working with such materials, two possible approaches are available.

According to a first approach, the double stranded nucleic acid is bound by means of a cationic polymer such as poly-L-lysine to the support (according to a technique disclosed in Fish and Ziff, Arth Rheum 24:534, 1981.

According to a second technique, the nucleic acid is bound covalently after the support itself has been activated. Various techniques are available for activating supports such as cellulosic paper for such purposes and such papers are now available commercially from various suppliers such as SCHLEICHER AND SCHUELL, or ORGENICS LTD.

One suggested technique for activating celluloses is the use of cyanuric chloride, which had previously not been adapted to flat surfaces such as paper and which is found to be most convenient and economical. This same method can be used for smoother surfaces such as celluloid, cellulose acetate, cellulose nitrate and agarose films.

Polysaccharides are similar to double-stranded nucleic acids in their inability to bind spontaneously to polymers. They can, however, be attached to plastics by means of another charged polymer, eg, poly-L-lysine. or they could be covalently bound to proteins by periodate oxidation, and Schiff's base formation, before attempting to absorb them onto plastics. Again, the cyanuric chloride activation technique may be used to bind polysaccharides, as well, to cellulosic materials.

Low molecular weight materials usually cannot be bound directly to a solid support and, therefore, must first be bound to a spacer molecule, eg, a protein, before binding to the solid support. United States Patent 4,299,916, the disclosure of which is herein incorporated by reference, reviews the various possibilities of binding a receptor to a solid phase support.

The receptors which are selectively attached at pre-arranged locations to the support will, quite obvviously, vary as a function of the analytes being assayed. Thus, for a given analyte, or group of analytes, the receptor selected will differ. Where the analyte is an antigen, the receptor would be the specific antibody. Where the analyte is an antibody, the receptor will be the corresponding antigen.

Once the receptor or plurality of receptors has been bound onto the support, such as by absorption and/or adsorption, particularly when the receptor is a protein, the support may be washed in a dilute detergent to prevent any further binding of protein. In certain instances, "blocking" or "saturation" of the surface by another protein in solution, unrelated to the bound protein, may be required such as is disclosed in USP 4,299,916.

Where the support surface has been chemically activated, inactivation by appropriate chemicals and under certain conditions may be required. For example, when cyanuric chloride-activated cellulose is used as the support, inactivation with 1M ethanolamine at pH 8.0 is preferred.

Depending upon the activity of the receptor, special precautions may be necessary to protect the receptor during storage prior to use. Additionally, depending upon the receptor, special drying techniques may be required.

With the receptor adhering onto the support, the analyte-containing sample can be applied to the support by various techniques, depending upon the quantities available, the source of the sample, and the shape of the solid support.

The treated substrate is contacted with the analyte-containing liquid for a sufficient time and under appropriate conditions to permit the various analytes to bind to their respective receptors. Depending on the circumstances, such application may take the form of dipping the substrate into a solution containing the analytes, swabbing the substrates with the solution, etc.

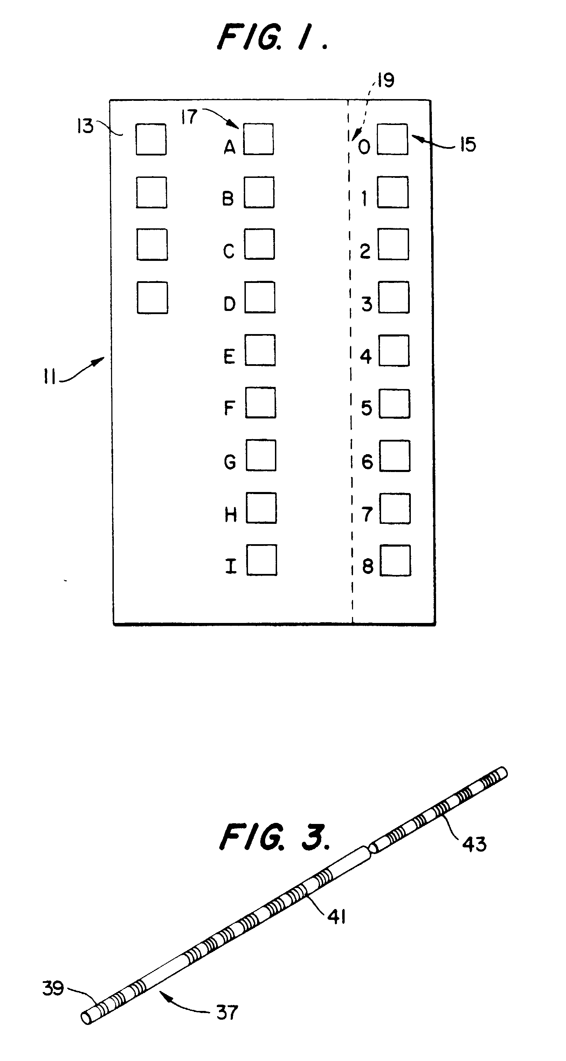

When using a card-like support 11 of the type shown in Figure 1, the entire card 11 may be dipped into the sample, if it is liquid, or the sample itself can be streaked or swabbed across the appropriate spaces on the solid support. The card is coated with control spots 13 for monitoring the various parameters of the assay and standard spots 15 which provide a standard curve for purposes of comparison with the receptor-treated areas 17.

Other analyte-receptor pairs may require other conditions. Solid or semi-solid samples such as baceterial cultures, tissues, etc, will require some degree of mechanical and/or enzymatic homogenization before applying them to the receptor-coated substrate surface.

During this stage of the procedure the physiochemical conditions for each analyte-receptor pair should be optimized. This means that for antigen-antibodyinteractions, for example, an isotonic environment with an above neutral pH would be ideal. Incorporation of the same detergent used to wash the solid phase after receptor binding may actually increase the specificity of the reaction and reduce unwanted background reactivity at later stages.

Of course, sufficient time must be allowed for interaction between the analyte and the receptor. The physiochemical conditions may be altered such that the contact time may be shortened, if desired, such as, for example, by higher temperature, or inclusion of some polymers in the interaction mixture, eg, 4% polyethylene glycol 6000.

Once bound onto the substrate receptors, the support is again washed to remove unwanted excess. The subsequent washing procedure is of utmost importance as is the case with any solid phase immuno-assay. This is particularly true of the multi-analyte system of the invention. The wash procedure may include a detergent, eg, BRIJ 35T, or another suitable detergent.

The wash procedure of the invention may be facilitated by utilizing a squeegy effect which wipes off excess liquid from the support.

The washed, analyte-containing support is then developed, to quantitatively and/or qualitatively establish the presence of the analytes. This may be achieved such as by development of a color reaction, a radioactive immunoassay, either competitive or non-competitive, a sandwich technique, etc.

The identity of the various analytes may be determined simply by observing their locations on the support since they will be adhering to receptors 17 which have been positioned at pre-determined locations on the support. Thus, the mere positive reaction at a given support location is indicative of the presence of a suspected analyte.

The washed support may be developed in the office, under field conditions, or in the laboratory.

The probe which is used to detect the bound analyte is normally a labelled receptor to the analyte in question. The probe will specifically attach to the receptor-bound analyte and allow detection and quantitation by virtue of a label association therewith.

In immunoassays for an antigen, the probe is preferably the same as the solid support-bound receptor. In immunoassays for antibodies where the solid support receptor is the antigen, the probe is preferably an antibody against the immunoglobulin-type of the analyte antibody, eg, labelled anti-human immunoglobulin would be the probe for human antibodies.

The probe antibodies may be labelled by radioactive isotopes or by enzymes by any of a number of well known techniques such as are described in United States Patents 3,654,090 and 4,099,916, the disclosures of which are hereby incorporated by reference thereto.

For purposes of the present invention, enzymes which form an insolubilized color reaction at the various probe locations such as peroxidase, glucose oxidase, or betagalactosidase may be used.

Although radioactive labels can alternatively be employed in the present invention this results in some complication and slowdown of the detection method. Thus, for purposes of the invention, enzymatic techniques are preferred.

In certain cases it is desirable to use an unlabelled probe, and then to detect the probe with a labelled material, eg, antibody to the probe. Such a technique may increase sensitivity at the expense of higher background reactions. This technique is also well known in the art, see for example, Hale and Randle, Biochem J 88:137, 1963, and need not be further described at this point.

### PROCEDURES

In the case of a double-stranded nucleic acid analyte bound to a support, one of three general approaches may be used to obtain a probe.

### Procedure I

Anti-double-stranded DNA (anti-dsDNA) antibodies are isolated from Systemic Lupus Erythematosus patients or animals with comparable diseases. Anti-single stranded DNA antibodies must first be removed by appropriate absorption techniques. Anti-dsDNA which remains can be labelled first and used directly to identify the analyte materials with which it binds to form a duplex. Alternatively, unlabelled duplex-bound anti-dsDNA antibodies may be detected subsequently with a labelled anti-immunoglobulin antibody while the duplex is bound onto the support.

### Procedure II

The analyte samples are first chemically modified so as to add an immunoreactive group to them without substantially damaging their stereo structure. One simple chemical modifier is picryl sulfonate which introduces a TNP (trinitrophenyl) group into the amines of the nucleic acid analyte. The production of anti-TNP antibodies is a well known procedure in the art, such as is disclosed in Nowotny, "Basic Exercises in Immunochemistry". Springer Verlag, New York, 1969. Another method for introducing an immunoreactive group into a nucleic acid is described by Poverenney et al, J Immunol Methods, 16:313, 1979. Labelled antibodies to the nucleic acid modifiers are then used as probes to detect the bound analytes.

### Procedure III

Nucleic acid molecules, identical to the receptor, may be labelled in vitro with immunoreactive groups such as described according to the second technique above, or by other techniques. These molecules are then mildly hydrolyzed to produce smaller pieces of nucleic acids which serve as probes. These probes are then allowed to anneal with parts of the bound analytes under mild denaturing conditions (60°C, 20-40% formamide). After annealing the probes are detected by antibodies to the immunoreactive groups as described immediately above.

When using enzyme labels, a color reaction is developed to detect the bound enzyme-labelled-probe. According to the invention, a preferred technique for developing an enzyme color reaction comprises using a peroxidase-labelled probe, or using any other enzyme which converts a soluble dye into an insoluble dye which precipitates at the site of the bound enzyme.

According to another alternative, the colorless dye may also be insoluble before conversion to develop a color reaction. The use of a dye which is capable of being rendered insoluble is extremely important according to the invention since the location of the dye must conform to the location of the enzyme-labelled antibody probe if accurate identification is to be made possible.

One known technique for the localization of peroxidase is described by Buckel and Zehelein (Gene 16:149, 1981) as part of an assay to detect a protein on the surface of bacterial colonies. The method involves touching a flat solid support (carrying the enzyme-labelled probe) to the surface of a gelatin-based gel, containing the substrate and dye system for peroxidase. While this method is applicable to the conditions of a research laboratory, it may not readily lend itself to a kit designed to be used under field conditions.

Thus, it is yet another aspect of the invention that the system of the invention may include an enzyme color development substrate system which is designed for improved storage and field use. Hydrogen peroxide, the substrate of the enzyme label, is extremely labile and will undergo spontaneous lysis when in aqueous solution. Therefore, the following substrate systems are provided according to the invention:
(a) A cellulose based sheet which may be made of paper, cellulose membrane, etc, and which is impregnated with a gelatin-based fluid gel, at high temperature, containing the dye and buffer, and allowed to dry spontaneously or by lyophilization. Before use, the sheet is dipped into a solution of hydrogen peroxide in 50% methanol and then touched to the peroxidase enzyme-carrying solid support for color development.
(b) The same technique is used as in (a) but the paper itself is also impregnated with glucose oxidase. Before use, the paper is dipped into a dilute aged glucose solution (2% concentration). Hydrogen peroxide will be generated by the glucose oxidase and will render the reagent kit more stable.
(c) The reagent mixture for the dye-substrate is completely dry and may comprise:
   (1) a gelling agent which requires no heating to be activated, eg, instant starch. sodium alginate;
   (2) a gelling initiator, where applicable, eg, calcium ions for alginate;
   (3) glucose oxidase and glucose (as a source for hydrogen peroxide);
   (4) a dye;
   (5) a buffer system; and
   (6) an effervescent (not required but may be used as a chemical means for improving mixing and facilitating reaction).

   Using this technique, the substrate mixture is suspended in an appropriate solvent such as water, or water and alcohol. The flat solid phase support is dipped into the suspension and immediately taken out. The color reaction will develop in the gel which adheres to the flat surface of the support. After development of the color, the gel may be washed or wiped off the solid support.
(d) This technique is the same as (c) except that potassium iodide is added rather than a dye. The iodide ion serves as an electron donor to the reaction catalyzed by the peroxidase. Molecular iodine thus formed binds to the starch, where this is the gelling agent, and forms a visible blue-colored complex. Quite obviously, each of the above detection techniques may also be used with a probe which is labelled with glucose oxidase. In this case, glucose and peroxidase should be included in all substrate-dye mixtures.

The method for attaching the substrate-dye mixture to the solid phase support described above may lend itself to other enzyme labels, making appropriate changes in substrate compositions.

An alternative dye generation system is described in Bio Rad Price List J. 1984 (p 169) and available commercially from that company or other suppliers of chemicals (eg Sigma). The system is based on 4-chloro-1-naphthol as the electron donor chromogenic substrate. In its reduced (colorless) form the material is soluble in alcohol:water mixtures. In the presence of peroxidase and hydrogen perioxide the material forms a blue-black precipitate. Using this technique, a probe which is labelled with peroxidase or glucose oxidase thereon can be detected by dipping the solid support on which the probe is affixed into a solution containing 4-chloro-1-naphthol and peroxide (in the case of a peroxidase labelled probe) or glucose and peroxidase (in the case of a glucose oxidase labelled probe).

### EXAMPLES

### Example 1:

### Potassium Iodide - Start Detection for Peroxidase

9.22 gr/liter monohydrate citric acid, 21.52 gr/liter Na₂HPO₄, are suspended in water and dissolved completely. 0.5-1.0 gr/liter potato starch or soluble starch is suspended in water. The starch is dissolved by boiling and cooling. The two solutions are mixed in 1:1 ratio. Potassium iodide is added until 3mM final, and toluene is added to 0.03% final as preservative, if needed. Filter papers may be soaked with this solution and dried to serve as indicator paper.

Prior to use the paper is dipped into hydrogen peroxide 2 x 10⁻⁴%. The re-hydrated paper may then be used by contacting it with a support having the probe affixed thereto whereby a chromogenic reaction appears on the paper.

Potato starch may be replaced by instant gelling starch at higher concentration (1-10% w/v of the total mixture depending on the material). In this case all components should be dry-mixed and hydrated just before dipping the solid support therein.

### Example 2:

### Chloro-Naphthol Chromogenic Mixture for Peroxidase

Mix 1 volume of 3 mg 4-chloro-1-naphthol per ml methanol with 5 volumes of a solution of 0.018% hydrogen peroxide in Tris buffered saline (20 mM Tris HCI, 500 mM NaCl. pH 7.5). This mixture provides a complete chromogenic mixture for peroxidase. Filter paper can be impregnated with the mixture as above and rehydrated prior to use as noted above.

### QUALITY CONTROL

A problem inherent in any immunological assay kit is the fact that it contains many reagents and involves many reactions, each of which may go wrong and yield a false-negative result. The present invention provides an optional system for monitoring some or all of the components of the assay for proper activity.

Additionally, the present invention provides for quantitative or semi-quantitative analysis of analyte presence using visual observation, or relatively inexpensive optical measurement apparatus.

Quantitative analysis of the various analytes is achieved by coating the support with a range of analyte concentrations for each suspected analyte, and then exposing these control regions of the substrate to the same conditions as the remaining portions of the substrate (subsequent to affixation of the sample analyte onto the support). By comparison of the results over the entire support it is possible to obtain a good semi-quantitative reading as to the presence of a particular analyte. This is done by comparing the color of the developed receptor regions with the developed regions of control analyte bound on the card. The control appears as spots 13 in a pre-designated area of the solid support (see Figure 1). Such a determination may be made by visual or instrument inspection. To facilitate comparison, perforations 19 are provided to establish a tear line on the card which allows for the designated area to be separated from the rest of the card, and be independently manipulated by the user.

### Antigen Analyte

Quality control may be provided by assaying while performing the following tests, each represented by a spot on the solid support:
(1) Control is provided for the activity of the dye-substrate system by means of enzyme (where an enzyme system is being used) bound individually to the support to ensure that the dye-substrate is properly operating.
(2) Control for the activity of the probe, ie, the enzyme-labelled antibody to the antigen analyte, is achieved by binding analyte to the solid support and observing the activity of the analyte upon exposure to the enzymatic probe-containing solution. A negative result indicates that the enzymatic probe solution may have lost activity. Alternatively, or in addition, the support may be coated in selected regions with avidin, and the enzyme-labelled probe is correspondingly labelled with biotin such that the probe can bind to the support. This should provide a positive result, upon chromogenic development, if the enzyme-dye substrate system is properly functioning.
(3) Control for establishing that all incubation times throughout the procedure have been sufficient is achieved by employing a non-related analyte-receptor system. The control analyate is either incorporated into the sample solution or is supplied in a separate container to be applied to the solid support at the time the sample is applied. The probe solution also contains probe for the control analyte such that non-cross-reacting control analyte reactions are subject to the same handlings and incubations as the analyte reactions which are the subject of the procedure.

### Antibody Analyte

The quality control system in an assay for an antibody analyte will include the following tests:
(1) A control for the activity of the dye-substrate system in the form of an enzyme bound to the solid support, eg, using the avidin-biotin technique referred to above.
(2) A control for the activity of the enzyme-labelled anti-immunoglobulin probe wherein immunoglobulin derived from the same species of the analyte antibody, is bound to the solid support surface.
(3) Control for sufficient incubation times is provided by a non-cross-reacting antigen bound to the solid support. If an anti-serum to the antigen, derived from the same species is available, then it may be included as a separate control solution or be incorporated into the sample-solution. Where an anti-serum is unavailable, as occurs with human samples, TNP-bovine serum Albumin may then be used as a support-bound antigen since for dietary reasons, many individuals carry antibodies to bovine antigens. Additionally, hydrophobic hapten TNP (trinitrophenyl) is able to bind a detectable amount of immunoglobulin from normal serum (See Fish and Ziff, J Immunol 128:409, 1982). In any case, batches of blood bank plasma or serum can be screened for the presence of such antibodies. Those demonstrating a high enough response may be incorporated as a separate control serum, or incorporated into the sample solution.

In both of the above examples, a slight problem exists as to control 1 in each case. The shelf life of the support-bound enzyme may be shorter than the enzyme in the probe. In that case, when the assay is being performed the control enzyme can be tagged with a biotin label which will affix the entire molecule to an avidin molecule, previously bound to the solid support. Thus, as part of the assay procedure the control enzyme may be affixed to the support, such that shelf storage ceases to be a problem.

### EXAMPLE 3:

### Control for the Dye-system:

Horseradish peroxidase conjugated to goat immunoglobulin (eg commercially available from Miles, or prepared according to Nakane and Kawasi, J Histochem Cytochem 22:1084, 1974) is diluted 1:100 in phosphate buffered saline pH 7.5 or in 1M sodium bicarbonate pH 9.6. The solution is applied to a polystyrene support surface.

After 30-60 minutes at room temperature (eg 25°C), the polystyrene is washed in 0.85% NaCI. The polystyrene support is then dipped in a 1% w/v solution of BSA (Bovine Serum Albumin CRG-7. Armour) for 30 minutes and washed first in 0.85% NaCI, and then in water. Alternatively, a solution of the pure enzyme itself (0.01-0.1 U/ml) in 0.15M ammonium bicarbonate containing 1% BSA is applied to the polystyrene surface and allowed to dry.

The support-bound enzyme is then exposed to the substrate-dye system.

### EXAMPLE 4:

### Control for the activity of the probe

When the analyte is an antibody, serum obtained from healthy young specimens of the same species producing the antibody analyte is diluted 1:1000 in phosphate buffered saline (PBS) or 1M sodium bicarbonate pH 9.6. The solution is applied to a polystyrene support, allowed to incubate for 30 minutes at room temperature, and washed off with 0.85% NaCI containing 0.1% BRIJ 35T.

Alternatively, immunoglobulin, isolated from whole serum can be employed. In this instance a 10 microgram/ml solution of the immunoglobulin in PBS or 1M sodium bicarbonate is used.

For an analyte which is an antigen, the control antigen is attached to the solid support in a manner similar to the above.
(a) With protein antigens, eg, immunoglobulin, bacterial toxins (pertussis toxin, cholera toxin, tetanus toxin), albumins, the antigens are diluted to 10 microgram/ml and applied to the surface.
(b) With nucleic acids and polysaccharides binding to plastic surfaces may be facilitated by a polycationic linking polymer such as poly-L-lysine. A suggested procedure is given in Fish and Ziff, Arth Rheum 24:534, 1981.

### EXAMPLE 5:

### Control for Sufficient Exposure Time and Proper Handling

(a) For an antigen analyte: As a non-cross-reactive antigen one of the following may be chosen: egg albumin, keyhole limpet hemocyanin (KLH) and any other antigen which is not related to the analyte-receptor system. An antiserum to the control antigen produced in the same animal species, in which the receptor-antibody to the tested analyte is being produced, is diluted 1:1000 in PBS or 1M sodium bicarbonate pH 9.6 and applied to the solid support at the appropriate space to serve as the receptor. The control analyte is added to the tested analyte or to the first wash solution at a concentration of 50-500 ng/ml. A control probe will contain an enzyme-labelled antibody to the control antigen. The control probe is added to the test probe solution at the same concentration.
(b) For an antibody analyte: one of the antigens above 5(a) is applied to the surface 10 µg/ml in PBS or 1M sodium bicarbonate pH 9.6) in the appropriate place. The antiserum to the control antiserum is added in a 1:1000 dilution to the measured analyte-antibody or to the first wash. The remainder of the procedure is then performed, as before.

### Nucleic Acids

When detecting an identifying nucleic acid sequences, the same type of controls may be employed, ie:
(1) Control for the substrate-dye system, as was previously described.
(2) Control for the labelled probe. A double-stranded nucleic acid or chemically modified nucleic acid can be attached to the solid support at the appropriate location.
(3) Control for hybridization time and conditions by using non-cross hybridizing nucleic acid pairs.

### STANDARD REFERENCE CURVE

The multi-analyte test described until this point may be used qualitatively. The system will thus provide a positive or negative answer as to the existence of a particular antigen, antibody or nucleic acid. However, "standard spots" can be added to the solid support to obtain quantitative information. The standard spots are all positioned to one side of a perforated tear line 19 (Figure 1).

In the semi-quantitative mode, each standard spot has a progressively increasing enzyme concentration and the standard spots will yield, after full development of the assay, a scale of color densities, each corresponding to a different amount and/or concentration of analyte. At the end of the procedure, the user can break or tear the solid support at the perforations (see Figure 1) and separate the standard panel from the analyte panel. This operation facilitates better comparison of the color-density between the analyte and the standard locations.

For more quantitative results, the entire control portion of the solid support may be scanned utilizing a suitable optical densitometer. An exact standard curve can then be constructed and the exact quantities of analyte can be more precisely determined.

Alternatively, or in addition, additional standard or control spots may be utilized, and will now be described.

### Procedure 1

A different amount of enzyme is attached at each spot directly on the support. After complete development with dye, each spot will demonstrate a different color density. The spots may be correlated by the manufacturer with different amounts of analyte and this information may be included with the test kit.

### Procedure 2

A different amount of avidin is attached at each spot. The enzyme-labelled probe is also tagged with biotin which will enable it to bind to the avidin on the standard spot. Afterward. the procedure is identical to that of Procedure 1, based upon a guide provided by the manufacturer.

### Procedure 3

For assay systems where the analyte is an antibody, the standard spots may contain different amounts of an immunoglobulin, derived from the same species, donating the antibody. The labelled probe (an anti-immunoglobulin) will then bind to these spots according to the amount of immunoglobulin attached and will yield different color densities. These densities are once again compared to a standard comparison chart provided by the manufacturer.

### Procedure 4

Receptor-analyte combinations may be employed as in Procedures 2 and 3. provided they do not interfere with the tested analytes or their receptors.

### Procedure 5

For an enzyme containing polysaccharide in its molecule (eg horseradish peroxidase) the plastic surface can be coated with different quantities of a lectin (a molecule which binds carbohydrates) such as Con A. The use of this approach, however, should be limited only to those cases where the analyte itself is devoid of carbohydrates.

### Procedure 6

Different concentrations of antibody produced against the enzyme, originating in an animal species identical to the donor of the antibody in the enzyme-antibody probe.

### EXAMPLES:

### 6. Example of Procedure 1

An enzyme-immunoglobulin conjugate prepared according to Nakane and Kawasi (J Histochem Cytochem 22:1084, 1974) is diluted 1:100, 1:200, 1:400, 1:800, 1:1600; 1:3200, in PBS pH 7.4 or in 1M sodium bicarbonate pH 9.6. Each dilution is applied on the plastic support at its predetermined spot. Following a 30-60 minutes incubation at room temperature. the whole plastic sheet is washed in 0.85% NaCI with 0.1% BRIJ 35T.

### 7. Example of Procedure 2

The antibody is labelled with biotinyl groups using commercially available reagents (eg from Sigma Chemical Co. St Louis, Mo, USA) or as described in the professional literature (Guedson, Ternyck and Avrameas, J Histochem Cytochem 27:1131, 1979). It is then conjugated to peroxidase by the method of Nakane and Kawasi, J Histochem 22:1084. Avidin obtained from commercial source (eg. Sigma) is dissolved to 10 micrograms/ml in PBS pH 7.4 or 1M sodium bicarbonate pH 9.6. This solution is serially diluted 1:1 to obtain the points of the standard curve. Each dilution is then applied onto the plastic support at its predetermined spot. Following a 30-60 minutes incubation, the plastic is washed as above.

### 7. Example for Procedure 3

Either one of the following two approaches can be employed:
(a) Using whole serum as the source of immunoglobulin; The unprocessed serum is diluted 1:1000 in PBS pH 7.4 or 1M sodium bicarbonate pH 9.6 and then is 1:3 serially diluted in the same buffer. The various concentrations are then applied as in Examples 6 and 7.
(b) Using separated immunoglobulin: immunoglobulin is derived from whole serum by ammonium sulphate precipitation (eg. Fish, Witz and Klein, Clin Exp Immunol 16:355, 1974). A solution of 10 microgram/ml in PBS pH 7.4 or 1M sodium bicarbonate pH 9.6 is prepared (immunoglobulin content is determined by OD at 280b and 260 nanometers as described by Layne, Methods Enzymology 3:447, Academic Press, 1966). The immunoglobulin solution is serially diluted 1:1 in the same buffers. The procedure is then continued as above with respect to Examples 6 and 7.

### 9. Example for Procedure 4

Basically similar to control procedure for sufficient exposure times and reaction conditions, employing non cross-reactive receptor analyte system (see above Example 5). However, in this case the receptors are reacted with various concentrations (eg, 20 ng/ml - 1000 ng/ml) of the analyte where the analyte is a protein antigen. If the analyte is an antibody, whole antiserum dilutions are placed on the solid support. The dilutions depend on the particular batch of antiserum and must be determined beforehand by testing serial 1:2 dilutions from 1:10 up to 1:10000.

### 10. Example for Procedure 5

Concanavallin A (obtainable from: Sigma, GIBCO, Miles) is dissolved to 10 micrograms/ml in PBS pH 7.4 or 1M sodium bicarbonate pH 9.6. it is then further serially diluted 1:2 in the same buffer. The individual dilutions are placed on the plastic support as described in Example 6.

### 11. Example for Procedure 6

Antiserum against the enzyme, eg horseradish peroxidase, is either obtained commercially or produced in the appropriate animal (rabbit, goat, chick) by repeated immunizations (an injection of 100 micrograms enzyme in 1 ml 0.85% NaCI emulsified with 1.0 ml complete Freund's adjuvant (GIBCO or Difco) injected is followed 3-4 weeks later with 200-500 µg peroxidase emulsified as above in incomplete Freund's adjuvant). The antiserum is applied to the plastic surface as described in procedure 3a above (Example 8).

### APPARATUS

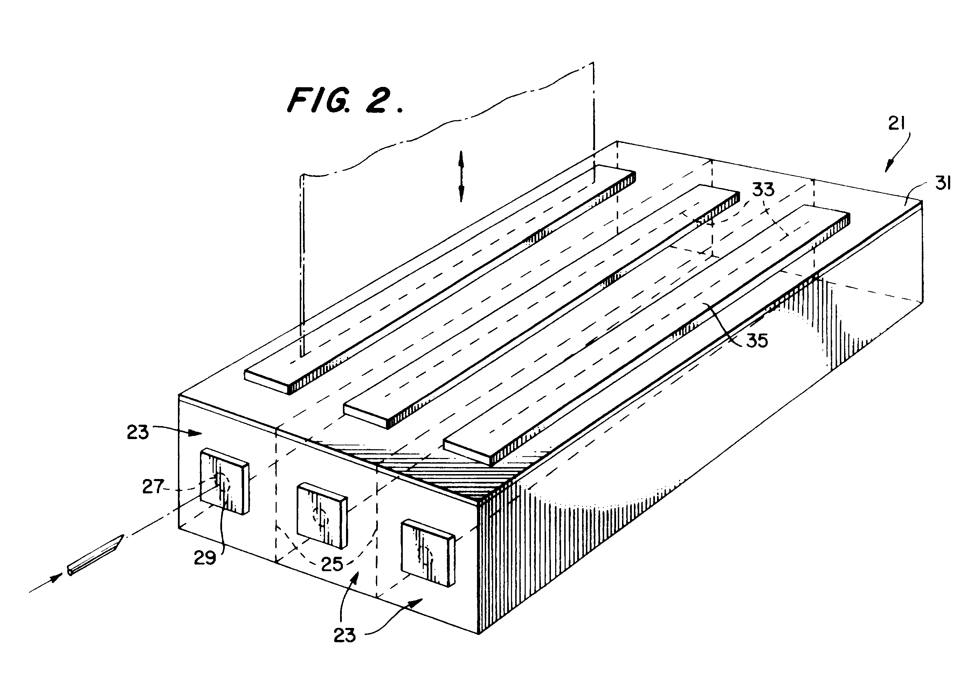

An assay according to the invention may be performed using a variety of apparatus. Since the various reagents are applied to a card. the assay may be performed in conjunction with apparatus of the type shown in Figure 2, which includes a compartment 21 having no lid which is divided into several compartments 23 by parallel inner walls 25. In one of the outside walls of each compartment, near the bottom of the vessel, an orifice 27, covered with a rubber septum 29, may be provided although this may not be necessary for all kinds of assays (this will depend on the solubility of the reagents). The entire vessel is covered with a flexible rubber sheet 31 having a longitudinal slit 33 in the center of each compartment which parallels the inner walls. The length of the slit should not be shorter than the width of the solid support. After the device has been loaded with the proper reagents in each compartment, the entire device is sealed with seals 35, such as, eg, with adhesive tapes. Each compartment is preferably sealed separately in a manner which allows for independent removal of the seal.

Such a device may be used in a manner which allocates each of the compartments to a given stage of the assay, such as, eg, incubation, wash, etc.

The reagents for each stage are loaded into the corresponding compartment and the compartments are arranged in the order they are used

Before use, the seal of each compartment is removed and a solvent is introduced into the compartment. The solid support is then dipped into the fresh solution for the required length of time. When incubation has been completed, the solid support is removed from the incubation mixture through the slit in the rubber cover. The elastic slit wipes the card and removes excess liquid from the solid surface.

When used in conjunction with a septum 27 at the bottom of each compartment. solvent may be injected forcefully into the dry reagents through the orifice. This technique may, in some cases, improve solubilization of certain reagents.

Although the invention has been described with reference to a flat card, it may likewise be adapted to other forms of solid support such as flat sticks or rods. Examples of one such embodiment are shown in Figure 3. In this embodiment rod 37 is provided with control spots 39, receptor spots 41, and standard spots 43 positioned on a separate portion of the rod. Other configurations can easily be imagined.

## Claims

1. A method for detecting at least one analyte in a single liquid solution suspected of containing more than one analyte, said method comprising the steps of:
(a) providing a solid support which is water insoluble and flexible and made from material selected from the group of polyvinyl, polystyrene, cellulose, nylon or glass, shaped in a form of flat, rectangular or round sheet, a rod, a stick or a cylinder;
(b) attaching and selectively positioning on said support at least one receptor for said at least one analyte, said receptor being selected from the group consisting of proteins, immuno- globins, nucleic acids or polysaccharides, carbohydrates, lipids;
(c) attaching a plurality of control spots on a pre-designated area of said solid support for monitoring a plurality of reactions of said analyte-detecting method;
(d) swabbing said liquid solution on said support (c), or dipping said support (c) into said liquid solution, wherein said liquid solution contains at least one analyte selected from the group consisting of proteins, nucleic acids, carbohydrates, immunoglobins, polysaccharides, lipids to form receptor-analyte conjugate reaction;
(e) contacting said receptor-analyte conjugate reaction (d) with a signal generating reagents to determine the presence and/or extent of said at least one analyte, wherein said signal generating reagents are labelled probes selected from the group consisting of antibodies, antigens, enzymes, carbohydrates, polysaccharides, antigens and nucleic acids, proteins and lipids;
said support further comprising standard spots thereon for establishing standard values which are compared with the results observed.

2. The method as claimed in claim 1 wherein said signal generating reagents produce a chromogenic reaction.

3. The method as claimed in claim 1 or claim 2 wherein said support is a flat sheet, and said sheet comprises a tear line, whereby a portion of said sheet may be separated from the remainder of said sheet.

4. The method as claimed in any one of claims 1 to 3 wherein said labelled probe is a receptor, whereby a sandwich is formed by said labelled probe, said analyte, and said receptor on said support.

5. The method as claimed in any one of the preceeding claims wherein said labelled probe is labelled with picryl sulfonate.

6. The method as claimed in any one of the preceeding claims wherein said support comprises a tear line to allow for separation of a portion of said support, and wherein said standard spots are all located to one side of said tear line whereby the portion of said support having said standard spots thereon may be separated from the remainder of said support and compared thereto.

7. The method as claimed in any one of the preceeding claims wherein said probe is monitored for activity.

8. The method as claimed in any one of the preceeding claims wherein said labelled probe is labelled with a member selected from the group consisting of an enzyme or radio active substance.

9. The method as claimed in claim 8 wherein said label is an enzyme, and said method further comprises contacting said support with a dye and substrate after exposure to said liquid solution and said labelled probe to provide a colour reaction permitting observation of the locations at which said analyte is bound.

10. The method as claimed in claim 9 comprising adding a gelling agent to said support and developing said colour reaction in said gel on said support.

11. The method as claimed in claim 9 comprising contacting said support with the developing sheet having a gel thereon, and developing said colour reaction on said developing sheet in said gel.

12. The method as claimed in claim 9 wherein said enzyme is selected from the group consisting of peroxidase, glucose oxidase and β-galactosidase,

13. The method as claimed in any one of the preceeding claims comprising amplifying the number of binding sites on said support prior to contacting said support with said receptors.

14. The method as claimed in any one of the preceeding claims wherein said support is a sheet which comprises a tear line to facilitate separation of portion of said sheet.

15. The method as claimed in any one of the preceeding claims comprising washing said support after contacting said liquid solution with said support to remove excess unbound material from said support.

16. The method as claimed in any one of the preceeding claims comprising providing a container having a plurality of compartments, the number of compartments corresponding to the number of steps performed, each compartment having a specific reagent or group of reagents therein, and performing said method by dipping said support within each of said compartments to contact said support with the reagent within each of said compartments.

17. An apparatus for performing said method according to any one of the preceeding claims comprising a container having a plurality of compartments therein, each of said compartments containing a reagent for said assay wherein each of said compartments is elongated and has an elongate opening at its top through which a card is inserted.

18. The apparatus as claimed in claim 17 wherein each of said elongated openings is covered with an elastic cover having an elongate slit therein to form two lips over each of said compartments through which a sheet may be inserted and removed with said lips serving to remove excess material from said sheet.

19. The apparatus as claimed in claim 18 wherein each of said compartments contains reagent in dehydrated form.

20. The apparatus as claimed in claim 19 wherein one wall of each said compartments has an orifice therein through which liquid may be injected to hydrate each of said reagents.

21. The apparatus as claimed in claim 20 wherein each of said orifices is covered with a sealing strip.

22. A support for performing said method according to any one of the preceeding claims whereby upon exposure to a liquid solution, and after subsequent processing, said support exhibits a standard reference curve which may be compared with another portion of said card for reference purposes and is formed by a series of discrete treated standard spots on said support, each of said standard spots exhibiting a different colour after exposure to said sample and final processing.

23. The support as claimed in claim 22 wherein said support is flexible card comprising a tear line thereon.

24. The support as claimed in claim 23 wherein all said standard spots are to one side of said tear line on a portion of said card whereby upon separation of said portion of said card along said tear line, said standard curve can be compared with the remainder of said card for reference purposes.

25. The support as claimed in claim 22 wherein said card has a plurality of receptors thereon adapted to bind more than a single analyte on separation location on said card.

26. The support as claimed in claim 22 wherein said support is made of a material selected from the group consisting bf: polyvinyl, polystyrene, cellulose, nylon, glass fibre, or mixtures thereof.

## Patentansprüche

1. Verfahren zur Bestimmung mindestens einer Analysensubstanz in einer einzelnen flüssigen Lösung, die im Verdacht steht, mehr als eine Analysensubstanz zu enthalten, wobei das Verfahren die folgenden Schritte umfaßt:
(a) Bereitstellen eines festen Trägers, der wasserunlöslich und flexibel ist, aus einem Material aus der Gruppe Polyvinyl, Polystyrol, Cellulose, Nylon oder Glas besteht und die Form eines flachen, rechteckigen oder runden Blattes, einer Stange, eines Stabes oder eines Zylinders besitzt;
(b) Anbringen und selektives Positionieren mindestens eines Rezeptors für die mindestens eine Analysensubstanz auf dem Träger, wobei der Rezeptor aus der Gruppe der Proteine, Immunoglobuline, Nukleinsäuren oder der Polysaccharide, Kohlenhydrate und Lipide ausgewählt wird;
(c) Aufbringen einer Mehrzahl von Kontrollflecken auf einer vorbestimmten Fläche des festen Trägers, um eine Mehrzahl von Reaktionen des Verfahrens zur Analysensubstanzbestimmung zu überwachen;
(d) Tüpfeln der flüssigen Lösung auf den Träger (c) oder Eintauchen des Trägers (c) in die flüssige Lösung, wobei diese flüssige Lösung mindestens eine Analysensubstanz enthält, die aus der Gruppe der Proteine, Nukleinsäuren, Kohlenhydrate, Immunoglobuline, Polysaccharide oder der Lipide ausgewählt wird, um eine Kopplungsreaktion zwischen Rezeptor und Analysensubstanz herbeizuführen;
(e) Zugabe eines signalerzeugenden Reagens zu dem Konjugat der Rezeptor-Analysensubstanz-Kopplungsreaktion (d), um das Vorhandensein und/oder den Gehalt der mindestens einen Analysensubstanz zu bestimmen, wobei das signalerzeugende Reagens eine markierte Sonde ist, die aus der aus Antikörpern, Antigenen, Enzymen, Kohlenhydraten, Polysacchariden, Antigenen und Nukleinsäuren, Proteinen und Lipiden bestehenden Gruppe ausgewählt ist;
wobei der Träger ferner Standardflecke zur Ermittlung von Standardwerten, die mit den festgestellten Ergebnissen verglichen werden, aufweist.

2. Verfahren nach Anspruch 1, dadurch gekennzeichnet, daß das signalerzeugende Reagens eine farberzeugende Reaktion hervorruft.

3. Verfahren nach Anspruch 1 oder 2, dadurch gekennzeichnet, daß der Träger ein flaches Blatt ist und eine Reißlinie besitzt, so daß ein Teil des Blattes von dem Rest des Blattes abgetrennt werden kann.

4. Verfahren nach einem der Ansprüche 1 bis 3, dadurch gekennzeichnet, daß die markierte Sonde ein Rezeptor ist, wodurch aus der markierten Sonde, der Analysensubstanz und dem auf dem Träger befindlichen Rezeptor ein Sandwich gebildet wird.

5. Verfahren nach einem der vorstehenden Ansprüche, dadurch gekennzeichnet, daß die markierte Sonde mit Pikrylsulfonat markiert ist.

6. Verfahren nach einem der vorstehenden Ansprüche, dadurch gekennzeichnet, daß der Träger eine Reißlinie aufweist, die eine Abtrennung eines Teils des Trägers gestattet, und daß sich die Standardflecke alle auf einer Seite dieser Reißlinie befinden, wodurch der Teil des Trägers, der die Standardflecke aufweist, von dem Rest des Trägers abgetrennt und mit diesem verglichen werden kann.

7. Verfahren nach einem der vorstehenden Ansprüche, dadurch gekennzeichnet, daß die Sonde auf ihre Aktivität überwacht wird.

8. Verfahren nach einem der vorstehenden Ansprüche, dadurch gekennzeichnet, daß die markierte Sonde mit einem Enzym oder mit einer radioaktiven Substanz markiert ist.

9. Verfahren nach Anspruch 8, dadurch gekennzeichnet, daß die Markierung ein Enzym ist und daß der Träger, nachdem er der flüssigen Lösung und der markierten Sonde ausgesetzt worden ist, zusätzlich mit einem Farbstoff und Substrat in Kontakt gebracht wird, um eine Farbreaktion hervorzurufen, die eine Beobachtung der Orte gestattet, an denen die Analysensubstanz gebunden ist.

10. Verfahren nach Anspruch 9, dadurch gekennzeichnet, daß ein gelbildendes Mittel auf den Träger gebracht und die Farbreaktion in dem Gel auf dem Träger entwickelt wird.

11. Verfahren nach Anspruch 9, dadurch gekennzeichnet, daß der Träger mit einem Entwicklerblatt, auf dem sich ein Gel befindet, zusammengebracht und die Farbreaktion auf dem Entwicklerblatt in dem Gel entwickelt wird.

12. Verfahren nach Anspruch 9, dadurch gekennzeichnet, daß das Enzym aus der Gruppe Peroxidase, Glucoseoxidase und β-Galactosidase ausgewählt wird.

13. Verfahren nach einem der vorstehenden Ansprüche, dadurch gekennzeichnet, daß die Zahl der Bindungsstellen auf dem Träger vor dem Zusammenbringen des Trägers mit den Rezeptoren vergrößert wird.

14. Verfahren nach einem der vorstehenden Ansprüche, dadurch gekennzeichnet, daß der Träger ein Blatt ist, das eine Reißlinie besitzt, die die Abtrennung eines Teils des Blattes erleichtert.

15. Verfahren nach einem der vorstehenden Ansprüche, dadurch gekennzeichnet, daß der Träger nach dem Zusammenbringen der flüssigen Lösung mit dem Träger gewaschen wird, um überschüssiges nichtgebundenes Material von dem Träger zu entfernen.

16. Verfahren nach einem der vorstehenden Ansprüche, dadurch gekennzeichnet, daß ein Behälter mit einer Vielzahl von Kammern bereitgestellt wird, wobei die Anzahl der Kammern der Zahl der durchgeführten Verfahrensschritte entspricht und jede Kammer ein spezifisches Reagens oder eine Gruppe von Reagenzien enthält, und daß der Träger in jeder dieser Kammern eingetaucht wird, um den Träger mit dem Reagens in jeder dieser Kammern in Kontakt zu bringen.

17. Vorrichtung zur Durchführung des Verfahrens gemäß einem der vorstehenden Ansprüche, gekennzeichnet durch einen Behälter mit einer Vielzahl von Kammern darin, wobei jede dieser Kammern ein Reagens für die Untersuchung enthält, jede der Kammern länglich ist und oben eine längliche Öffnung besitzt, durch die eine Karte eingeführt werden kann.

18. Vorrichtung nach Anspruch 17, dadurch gekennzeichnet, daß jede der länglichen Öffnungen mit einer elastischen Abdeckung abgedeckt ist, in der sich ein länglicher Schlitz befindet, so daß zwei Lippen über jeder Kammer ausgebildet werden, durch die ein Blatt eingeführt und herausgenommen werden kann, wobei die Lippen dazu dienen, überschüssiges Material von dem Blatt zu entfernen.

19. Vorrichtung nach Anspruch 18, dadurch gekennzeichnet, daß jede der Kammern des Reagens in dehydratisierter Form enthält.

20. Vorrichtung nach Anspruch 19, dadurch gekennzeichnet, daß jeweils eine Wand jeder Kammer eine Öffnung aufweist, durch die Flüssigkeit eingespritzt werden kann, um jedes der Reagenzien zu hydratisieren.

21. Vorrichtung nach Anspruch 20, dadurch gekennzeichnet, daß jede der Öffnungen mit einem Dichtungsband abgedeckt ist.

22. Träger zur Durchführung des Verfahrens gemäß einem der vorstehenden Ansprüche, dadurch gekennzeichnet, daß er nach der Behandlung mit einer flüssigen Lösung und nach Durchführung der nachfolgenden Verfahrensschritte eine Standardvergleichskurve zeigt, die zu Vergleichszwecken mit einem anderen Teil der Karten verglichen werden kann und die durch eine Reihe von einzelnen, behandelten Standardflecken auf dem Träger gebildet wird, wobei jeder der Standardflecken eine verschiedene Farbe zeigt, nachdem er der Probe ausgesetzt und endbehandelt wurde.

23. Träger nach Anspruch 22, dadurch gekennzeichnet, daß der Träger eine flexible Karte ist, auf der sich eine Reißlinie befindet.

24. Träger nach Anspruch 23, dadurch gekennzeichnet, daß sich alle Standardflecke auf einer Seite der Reißlinie auf einem Teil der Karte befinden, wodurch nach Abtrennung dieses Teils der Karte entlang der Reißlinie die Standardkurve zu Vergleichszwecken mit dem Rest der Karte verglichen werden kann.

25. Träger nach Anspruch 22, dadurch gekennzeichnet, daß auf der Karte eine Mehrzahl von Rezeptoren vorhanden ist, die mehr als eine einzige Analysensubstanz auf getrennten Orten der Karte zu binden vermögen.

26. Träger nach Anspruch 22, dadurch gekennzeichnet, daß der Träger aus einem Material besteht, welches aus der aus Polyvinyl, Polystyrol, Cellulose, Nylon, Glasfaser oder deren Gemischen bestehenden Gruppe ausgewählt ist.

## Revendications

1. Procédé pour détecter au moins un analyte dans une seule solution liquide, susceptible de contenir plus d'un analyte, ledit procédé comprenant les étapes suivantes:
(a) fourniture d'un support solide, insoluble dans l'eau et flexible, composé d'un matériau sélectionné dans le groupe comprenant le polyvinyle, le polystyrène, la cellulose, le nylon ou le verre, configuré sous forme d'une feuille plate, rectangulaire ou ronde, d'une tige, d'un bâton ou d'un cylindre;
(b) fixation et positionnement sélectif sur ledit support d'au moins un récepteur pour ledit au moins un analyte, ledit récepteur étant sélectionné dans le groupe constitué des protéines, des immunoglobulines, des acides nucléiques ou des polysaccharides, des glucides, des lipides;
(c) fixation de plusieurs points de contrôle sur une surface prédéfinie du dit support solide en vue de contrôler plusieurs réactions dudit procédé de détection d'un analyte;
(d) badigeonnage de ladite solution liquide sur ledit support (c) ou immersion du dit support (c) dans ladite solution liquide, ladite solution liquide contenant au moins un analyte choisi dans le groupe constitué des protéines, des acides nucléiques, des glucides, des immunoglobulines, des polysaccharides, des lipides, en vue de former une réaction conjuguée récepteur-analyte;
(e) mise en contact de ladite réaction conjuguée récepteur-analyte (d) avec un réactif générateur de signaux pour déterminer la présence et/ou l'étendue du dit au moins un analyte, lesdits réactifs générateurs de signaux étant des échantillons marqués choisis dans le groupe constitué des anticorps, des antigènes, des enzymes, des glucides, des polysaccharides, des antigènes et des acides nucléiques, des protéines et des lipides;
ledit support comprenant en outre des points de référence pour établir des valeurs de référence qui sont comparées avec les résultats observés.

2. Procédé selon la revendication 1, dans lequel lesdits réactifs générateurs de signaux entraînent une réaction chromogénique.

3. Procédé selon la revendication 1 ou la revendication 2, dans lequel ledit support est une feuille plate, et dans lequel ladite feuille comporte une ligne de déchirure, une partie de ladite feuille pouvant ainsi être séparée du restant de ladite feuille.

4. Procédé selon l'une quelconque des revendications 1 à 3, dans lequel ledit échantillon marqué est un récepteur, un sandwich étant ainsi formé par ledit échantillon marqué, ledit analyte et ledit récepteur sur ledit support.

5. Procédé selon l'une quelconque des revendications précédentes dans lequel ledit échantillon marqué est marqué avec du sulfonate de picryle.

6. Procédé selon l'une quelconque des revendications précédentes, dans lequel ledit support comprend une ligne de déchirure permettant la séparation d'une partie du dit support, et dans lequel lesdits points de référence sont tous situés sur un côté de ladite ligne de déchirure, la partie du dit support comportant lesdits points de référence pouvant ainsi être séparée du restant du dit support et y être comparée.

7. Procédé selon l'une quelconque des revendications précédentes, dans lequel on contrôle l'échantillon du point de vue de son activité.

8. Procédé selon l'une quelconque des revendications précédentes, dans lequel ledit échantillon marqué est marqué avec un membre sélectionné dans le groupe comprenant une enzyme ou une substance radioactive.

9. Procédé selon la revendication 8, dans lequel ledit marqueur est une enzyme, ledit procédé comprenant en outre la mise en contact du dit support avec un colorant et un substrat après exposition à ladite solution liquide et au dit échantillon marqué pour déclencher une réaction colorée permettant d'observer les emplacements auxquels ledit analyte est lié.

10. Procédé selon la revendication 9, comprenant l'addition d'un gélifiant au dit support et le développement de ladite réaction colorée dans ledit gel sur ledit support.

11. Procédé selon la revendication 9, comprenant la mise en contact du dit support avec la feuille de développement, comportant un gel, et le développement de ladite réaction colorée sur ladite feuille de développement dans ledit gel.

12. Procédé selon la revendication 9, dans lequel ladite enzyme est sélectionnée dans le groupe comprenant la peroxydase, la glucose oxydase et la β-galactosidase.

13. Procédé selon l'une quelconque des revendications précédentes comprenant l'amplification du nombre des sites de liaison sur ledit support avant la mise en contact du dit support avec lesdits récepteurs.

14. Procédé selon l'une quelconque des revendications précédentes, dans lequel ledit support est une feuille comportant une ligne de déchirure pour faciliter la séparation d'une partie de ladite feuille.

15. Procédé selon l'une quelconque des revendications précédentes, comprenant le lavage du dit support après la mise en contact de ladite solution liquide avec ledit support pour éliminer le matériau non lié excédentaire du dit support.

16. Procédé selon l'une quelconque des revendications précédentes, comprenant la fourniture d'un récipient comportant plusieurs compartiments, le nombre des compartiments correspondant au nombre d'étapes effectuées, chaque compartiment contenant un réactif spécifique ou un groupe de réactifs, ainsi que l'exécution du dit procédé par immersion du dit support dans chacun des dits compartiments en vue de la mise en contact du dit support avec le réactif contenu dans chacun des dits compartiments.

17. Appareil pour l'exécution du dit procédé selon l'une quelconque des revendications précédentes, comprenant un récipient à plusieurs compartiments, chacun des dits compartiments contenant un réactif pour ledit essai, dans lequel chacun des dits compartiments est allongé et comporte une ouverture allongée sur sa partie supérieure à travers laquelle une carte est insérée.

18. Appareil selon la revendication 17, dans lequel chacune des ouvertures allongées est recouverte d'une couverture élastique, comportant une fente allongée pour former deux bords au-dessus de chacun des dits compartiments, à travers lesquels une feuille peut être insérée et retirée, lesdits bords servant à éliminer le matériau excédentaire de ladite feuille.

19. Appareil selon la revendication 18, dans lequel chacun des dits compartiments contient un réactif sous forme déshydratée.

20. Appareil selon la revendication 19, dans lequel une paroi de chacun des dits compartiments comporte un orifice, à travers lequel un liquide peut être injecté en vue de l'hydratation de chacun des dits réactifs.

21. Appareil selon la revendication 20, dans lequel chacun des dits orifices est recouvert d'une bande d'étanchéité.

22. Support pour l'exécution du dit procédé selon l'une quelconque des revendications précédentes, ledit support présentant, lors de l'exposition à une solution liquide, et après traitement ultérieur, une courbe de référence, pouvant être comparée avec une autre partie de ladite carte pour des objectifs de référence, et étant formée par une série de points de références à traitement discret sur ledit support, chacun des dits points de référence présentant une couleur différente après exposition au dit échantillon et après traitement final.

23. Support selon la revendication 22, dans lequel ledit support est composé d'une carte flexible, comportant une ligne de déchirure.

24. Support selon la revendication 23, dans lequel tous lesdits points de référence sont tous situés sur un côté de ladite ligne de déchirure, sur une partie de ladite carte, la séparation de ladite partie de ladite carte le long de ladite ligne de déchirure permettant ainsi de comparer ladite courbe de référence avec le restant de ladite carte pour des objectifs de référence.

25. Support selon la revendication 22, dans lequel ladite carte comporte plusieurs récepteurs, pouvant lier plus d'un seul analyte à l'emplacement de la séparation sur ladite carte.

26. Support selon la revendication 22, dans laquelle ledit support est fait en un matériau sélectionné dans le groupe comprenant: le polyvinyle, le polystyrène, la cellulose, le nylon, la fibre de verre ou des mélanges de ces composants.
